(19) Europäisches Patentamt
European Patent Office
Office européen des brevets

(11) **EP 1 279 727 B1**

(12) **EUROPEAN PATENT SPECIFICATION**

(45) Date of publication and mention
of the grant of the patent:
**01.07.2009 Bulletin 2009/27**

(51) Int Cl.:
*C12N 1/20* *(2006.01)*    *A61K 36/00* *(2006.01)*

(21) Application number: **01947108.5**

(22) Date of filing: **26.04.2001**

(86) International application number:
**PCT/CN2001/000616**

(87) International publication number:
**WO 2002/012446 (14.02.2002 Gazette 2002/07)**

(54) **PROCESS FOR PRODUCING PREPARATIONS OF NATURAL MEDICINES**

VERFAHREN ZUR PRODUKTION VON NATÜRLICHEN MEDIZIN PRÄPARATEN

PROCEDE POUR LA PRODUCTION DE PREPARATIONS DE MEDECINES NATURELLES

(84) Designated Contracting States:
**DE GB SE**

(30) Priority: **26.04.2000 CN 00111083**

(43) Date of publication of application:
**29.01.2003 Bulletin 2003/05**

(73) Proprietor: **Wu, Bingxin**
**Jinan City**
**Shandong 250100 (CN)**

(72) Inventors:
• **WU, Bingxin**
**Bingxin Wu**
**Jinan City,**
**Shandong 250100 (CN)**
• **DONG, Lishan**
**Bingxin Wu**
**Jinan City,**
**Shandong 250100 (CN)**

• **SUN, Xiaolin**
**Bingxin Wu**
**Jinan City,**
**Shandong 250100 (CN)**
• **WANG, Hong**
**Bingxin Wu**
**Jinan City,**
**Shandong 250100 (CN)**

(74) Representative: **Sajda, Wolf E. et al**
**Meissner, Bolte & Partner GbR**
**Widenmayerstrasse 48**
**80538 München (DE)**

(56) References cited:
**CN-A- 1 144 114**

• **DATABASE WPI Section Ch, Week 200010**
**Derwent Publications Ltd., London, GB; Class**
**B04, AN 2000-106942 XP002287449 & CN 1 232**
**682 A (JIA Y) 27 October 1999 (1999-10-27)**

**Description**

Field of Invention

The present invention relates to a process for producing preparations of natural medicines (i.e., Chinese traditional herbs). Particularly, the present invention relates to a process for bio-conversion or bio-modification of any plant or animal original natural medicines by the intestinal probiotics fermentation culture which mimic the micro-ecosystem in human intestinal tract in the light of the bionics theory, whereby the biological activity of said natural medicines is improved and toxicity thereof is reduced or eliminated. The present invention further relates to the use of said process in producing natural Chinese herbal preparations.

Background of Invention

Chinese herbal medicines have been used in the clinical in Eastern country, particularly in China, for thousands of years, although the exact mechanism of the Chinese herbal medicine, say, pharmacology effect is not well known yet. With the development of the study on human microecosystem, it is come to believe that' the microecology alteration of human intestinal microorganisms may be closely related to the "disease" in view of Chinese traditional medical science. Specifically, the modulation using Chinese herbal medicine may improve the balance between those beneficial bacteria and those harmful bacteria in the intestinal tract. On another hand, the balanced intestinal microeco- enviroment will facilitate the absorption, conversion, and transportation of the natural medicines and thereby improve the biological effect thereof.

The efficiency of Chinese herbal medicine relies on the chemical material contained therein. When administrated orally into a human body in the form of decoction liquor in the sense of Chinese traditional medicals, i.e., water extract, it will finally be absorbed by various transportation routes: (1) antetype compounds are absorbed in to circulation and exhibit its pharmacological effect; (2) antetype compound is absorbed and then is further subjected a series of procession in hepatic-intestinal circulation, in tissues or in bloods before exerting its pharmacological effect; (3) antetype compounds are not absorbed and discharged with excrement and urine; (4) antetype compounds are chemically converted by intestinal bacteria metabolic enzymes as the result of normal intestinal bacteria's activity.

In this way, the absorption rate, transportation rate and biological activity of said medicines are improved accompanied with reduced toxic side effect, which is favorable to exhibit its pharmacological effect. Thus, it is very important for illustrating the functional mechanism of Chinese traditional medicine to fully investigate the exact process of natural medicines by human intestinal anaerobes and metabolic conversion thereof with respect to pharmacokinetics study in laboratories as well as clinical trails.

Since 1950's, various animal models and methods had been used to investigate the conversion with respect to natural medicines or active components thereof and structural modification thereon by intestinal normal flora. It has been proved by studies *in vivo* or *in vitro* that there is a complex metabolic process for bio-conversion and structural modification by intestinal normal flora to natural medicines administrated in the form of decoction liquor (i.e. water extract), which plays an important role in the absorption, transportation, metabolite and efficiency *in vivo* for natural medicines.

For example, Dooth A.N. et al.,(J. Biol.Chem., 223:251. 1956), had reported that B cycle degradation product, such as 3,4-dihydroxy-phenylacetate, could be detected in the urine after orally administrating quercetin to mouse. On the contrary, these quercetin metabolic products could not be detected in urine if antibiotic (such as neomycin) is administrated previously to the animals. The experimental results suggest that these metabolic products are brought by the intestinal bacteria metabolic conversion.

Drasar and Hill (Human Intestinal Flora, Academic press, 1974) further proved that once rat was Intraabdominally administrated antibiotics to suppress intestinal bacteria, no toxic response to almond glycoside in rat can be observed when orally administrating Chinese herb, almond that contains highly toxic almond glycoside, to experimental rat. In addition, it is shown that if almond glycoside is incubated with isolated intestinal bacteria of mouse, said almond glycoside can be hydrolyzed into phenylethanolnitrile and in turn, it is further degraded into toxic hydrocyanic acid. (see, Scheline R.K., Pharmacol. Rev., 25:451, 1973).

It is also proved that the main components of glycyrrhizia, liquirtin, can be converted into enoxolone by Eubacterium sp. CLH with the aid of its products, glucuronidase. In addition, it was observed that the plants derived isoflavones compound from leguminous species can be structural modified by incubation with intestinal content from caprine. Particularly, 7-hydroxy-4-methylisoflavone is converted into soybean glycoside ligand and epuol with biochanin A converting into dye genitein. That is to say, the intestinal flora in the content of carprine have the ability to bio-convert isoflavone compounds. (see, Niissou, A., et al., Biochem. Biophys. Acta, 148:92, 1967).

The document CN 1232682 (JIA Y) discloses a process for treating natural medicine from animal source by comestible yeast. In this conventional technology, the fermentation process is carried out under aerobic conditions. The document is silent as to any intestinal probiotics.

All the above experiments, among others, imply that when intake into body via gastro-intestinal track, natural medicaments with known and/or unknown structure have to be structural modified to a certain extent by the action of intestinal flora or metabolites thereof before its absorption and then in function of its biological activity.

However, so far there is no such report in the prior art in relation to the process or treatment *in vitro* on natural medicines with unknown structure, especially natural medicine compositions by making use of known probiotics to activate, induce, convert and/or modify the active component thereof, thereby the biological functions are enhanced after oral administration. Moreover, there is no report to be found on the process of natural medicine preparation by fermentation *in vitro* by inoculating in batches of probiotics to improve the biological activity of said medicine before successfully applying the resultant in the clinic.

The present inventor, on the basis of long-period effort in the development of the intestinal probiotics preparation as well as anti-cancer natural medicines, has fermented various intestinal probiotics in combination with water extracts of various natural medicine compositions, wherein intestinal probiotics used in the present invention are widely distributed in the human body and possess exact activity. It is found surprisingly that the pharmacological activity *in vivo* of said natural medicine compositions have been substantially enhanced after the fermentation treatment by

intestinal probiotics and metabolites thereof. The present invention is achieved based on the above discovery.

Summary of Invention

One object of the present invention is to provide a process for producing natural medicine preparation, comprising inoculating in batches one or more intestinal probiotics into a culture medium which contains the extract of said medicines; and culturing the same under suitable fermentation conditions, wherein the intestinal probiotics are selected from the group of Bifidobacterium, Lactobacillus, Streptococcus, Enterococcus, Propionium, Leuconostoc, and Bacillus.

In a preferred embodiment of the present invention, said process comprise the steps of preparing a co-fermentation culture of one or more intestinal probiotics; and then adding into said culture medium a pre-made extract of one or more natural medicines or the powder of non-processed natural medicines; then further mixing and culturing the resultant mixture.

In another preferred embodiment of the present invention, said process comprises the steps of (a) adding into the suitable culture medium a pre-made extract of one or more natural medicines or the powder of non-processed natural medicines; (b) subsequently inoculating in batches one or more intestinal probiotics into the mixture obtained in step (a) and culturing the same by co-fermentation.

In a further preferred embodiment of the present invention, said probiotics are one or more human intestinal non-pathogenic probiotics that are selected from the species of the group consisting of *Bifidobacterium, Lactobacillus, Streptococcus, Enterococcus, Propionium, Leuconostoc*, and *Bacillus.*

In a further preferred embodiment of the present invention, said probiotics are selected from the species of the group consisting of *Bifidobacterium bifidum, Bifidobacterium breve, Bifidobacterium infantis, Bifidobacterium longum, Lactobacillus acidophilus, Lactobacillus bulgaricus, Lactobacillus bulgaricus, Lactobacillus casei, Lactobacillus delbrueckii, Lactobacillus plantarum, Streptococcus thermophilus, Enterococcus faecalis, Enterococcus faecium, Leuconostoc citrovorum, Propionibacterium shermanii*, *Bacillus subtilis,* and *Bacillus licheniformis.*

In a preferred embodiment of the present invention, the preparations of natural medicines comprise one or more of any plant or animal originated medicines or combinations thereof having prophylaxis and/or treatment effect and which can be processed or modified by one or more human normally intestinal bacteria and metabolites thereof.

In a preferred embodiment of the present invention, the preparations of natural medicines are the water-extract or organic-solution extract of one or more natural medicines, or the non-processed solid powders of said medicines.

In a preferred embodiment of the present invention, the organic solution is selected from the group consisting of methanol, ethanol, n-propanol, isopropanol, methylether, ethyl ether, dimethylether, acetone, dichloromethane, chloroform, acetonitrile, or the mixture thereof.

In a preferred embodiment of the present invention, the preparations of natural medicines are selected from the group consisting of medicaments for eliminating pathogenic factor for supporting vital QI; hemostasis by invigorating QI; invigorating the spleen and tonifying the kidney; softening the hard mass and removing blood stasis; resolving phlegm and eliminating dampness; relieving inflammation and evacuating toxin; promoting generation of blood; promoting diuresis and invigorating vital QI; or selected from a mixture of such medicaments. The terms, such as "QI" which means vital energy or functional activity of human body, "spleen", and "kidney", etc., used herein are all the concepts of Chinese traditional medical science and is clear to the person skilled in the art of said field.

Details of Invention

The present invention discloses a process for producing preparations of natural medicines as defined in claim 1. Specific embodiments thereof are defined in the subclaims. Particularly, the specification discloses a process for bio-conversion or bio-modification of any plant or animal original natural medicines by the intestinal probiotics co-fermentation culture which mimic the microecosystem in human intestinal tract in the light of the bionics theory, whereby the biological activity of said natural medicaments is improved and toxicity thereof is reduced or eliminated. The specification further discloses the use of said process in producing of natural medicine preparation.

It is well known that water decoction of natural Chinese traditional herbs is the widest-used crude medicines processing method during the procession of Chinese traditional medicine through history. However, water decoction process (i.e., process for decoction of Chinese traditional medicines) is simple but has inherent defects. In particular, the water-soluble components in the medicines fail to dissolve sufficiently, while most of lipo-soluble components therein are discarded during such a process. In recent decades, modem pharmaceutics production techniques for Western medicines, such as ethanol or other organic solution extract techniques, have been introduced and used in combination with the traditional water extraction. Unfortunately, some problems, such as remaining of organic solution, bad absorption and poor activity of medicines, are incurred when using these methods.

In the 1990's, it was proposed to produce Chinese traditional medicine using so called "semi-bionic extraction" which aims to mimic the gastrointestinal transportation procedure of oral administrated medicine, wherein the active components in Chinese herbs are extracted with acidic aqueous solutions and basic aqueous solutions, respectively, to facilitate absorption in the small intestine. However, the inner environment of the human gastro-intestine is unsuitably simplified and no consideration is made of the complexity of the components in Chinese herbs *per se*.

In fact, the human gastro-intestine is a micro ecosystem comprising about 100 kinds of beneficial or harmful bacteria in a number of more than that of human cells totally. In addition, each formula of Chinese herbal medicine comprises not a single compound as that of a chemically synthesised drug but almost a complex "reservoir" including various different chemical materials. Therefore, processing Chinese traditional medicine by simply mimicking the acid and/or basic condition in the gastro-intestine can hardly achieve the expected effect on that of processed Chinese traditional medicines.

The present inventor, based on the long-historic practice of development microecological preparation and Chinese herb preparation, has found that the process of the present application to said natural medicine preparation would-substantially improve the expected pharmacological activity *in vivo* of the same and reduce non-specific physical toxicity thereof in some extent. Said process comprises the steps of manipulating various natural medicines by selecting intestinal probiotics cultures, adding three kinds of probiotics into pre-made water decoction preparation of Chinese herbal medicines, and then co-culturing the resultant mixture.

One aspect of the present disclosure is to provide a process for bio-conversion and/or bio-modification of any plant- or animal- derived natural medicinces by intestinal probiotics co-fermentation culture, which mimic the micro-ecosystem in the human intestinal tract in the light of bionics theory, whereby the biological activity of said natural medicines is improved and toxicity thereof is reduced or eliminated. Said process includes the following steps of

(1) preparing the extracts of plant- or animal- derived natural medicine according to a conventional method, respectively, then condensed under reduced pressure before mixing the resultant;
(2) inoculating in batches the selected probiotics sequentially into the fermenter which contains the extract of natural medicament of step, (1); and culturing the resultant mixture under suitable fermentation condition; and
(3) when fermentation is finished, optionally, heating or not heating the fermentation culture to kill probiotics therein before collecting the culture.

Said probiotics according to the present invention may be one or more of any inherent beneficial, non-toxic bacteria in human intestinal, which can be selected from the group consisting *of Bifidobacterium, Lactobacillus, Streptococcus, Enterococcus, Propionium, Leuconostoc,* and *Bacillus.*

By way of example, the preferred strains include but are not limited to, species of the group consisting of *Bifidobacterium bifidum, Bifidobacterium breve, Bifidobacterium infantis, Bifidobacterium longum, Lactobacillus acidophilus, Lactobacillus bulgaricus, Lactobacillus bulgaricus, Lactobacillus casei, Lactobacillus delbrueckii, Lactobacillus plantarum, Streptococcus thermophilus, Enterococcus faecalis, Enterococcus faecium, Leuconostoc citrovorum, Propionibacterium shermanii, Bacillus subtilis,* and *Bacillus licheniformis.* For the purpose of the present invention, *Bifidobacterium* including *Bifidobacterium bifidum, Bifidobacterium breve, Bifidobacterium infantis, Streptococcus thermophilus,* and *Enterococcus faecalis,* are preferable.

Typically, there is no particular limitation to the selection of the species or strains of probiotics. Taking bionics and ecology balance into consideration, preferably, two or more (up to 9 strains) probiotics are selected which colonize at different locations in the human intestine and are complementary in function to each other. In one example, a group of three kinds of probiotics comprising *Lactobacillus acidophilus, Bifidobacterium* including *Bifidobacterium bifidum, Bifidobacterium breve,* and *Enterococcus faecium,* is used in the present invention.

It is well known that *Lactobacillus acidophilus, Bifidobacterium* (including *Bifidobacterium bifidum, Bifidobacterium breve*), and *Enterococcus faecium,* all belong to the normal intestinal flora. They are located at different portions in the intestine and have preferential functions at particular portions in the small intestine and colon, respectively. Particularly, *Lactobacillus acidophilus* is preferentially located in ileum which synthesises vitamins in the intestine, assists food digestion, facilitates metabolic in the host and enhances the lactose tolerance of the host. As to *Bifidobacterium,* it mainly colonizes in the colon and facilitates, as the most important physiological bacteria, the nutrition intake, growth and development, biological aversion and immunology function in the host. For *Enterococcus faecium,* it is predominantly found in caecum and exhibits function in potentially reducing the cholesterol level in the serum. That is to say, these preferred three probiotics of the present invention are widely distributed in the human body and have substantial physiological activity. It is held by the present inventor that the selection of the above-described three probiotics is completely enlighten by the bionics theory that serves as the basis of the present invention.

Alternatively, to facilitate the survival of said probiotics, such a group of three probiotics may also be used which include *Lactobacillus acidophilus, Propionibacterium shermanii,* and *Leuconostoc citrovorum.* In particular, (1) excessive lactate produced by *Lactobacillus acidophilus* in the intestine can be converted into propanic acid and carbon dioxide by, *Propionibacterium,* thereby the growth of molds and yeasts is prevented (2) alcohol dehydrogenase for hydrolyzing acetaldehyde, a harmful metabolite brought by the growth of yeast in the gut, can be synthesized by *Propionibacterium shermani*; (3) *Leuconostoc citrovorum* can assimilate simple sugar and citrate and produce carbon dioxide that is beneficial to the growth of *Lactobacillus acidophilus* and *Propionibacterium shermani.*

The Chinese traditional herbal compositions used herein refer to a medicament with more than one plant- or animal- origin Chinese herbs, and said Chinese herbs may be crude drugs with unmodified basic tissue structure, or water-extract or organic solution extract thereof. The organic solution used herein includes, but is not limit to, alcohols, such as methanol, ethanol, n-propanol, isopropanol, n-butanol, isobutanol; ethers, such as methylether, ethyl ether, dimethylether; acetone; dichloromethane; chloroform; acetonitrile; ethyl formate, ethyl acetate or the mixture thereof. Preferably, said Chinese herb is a water extract of natural medicine in view of the environment safety and fermentation manipulation.

In a point of pharmacological function, said natural medicine compositions that can be treated by fermentation *in vitro* according to the present disclosure include, but are not limit to, medicaments for eliminating pathogenic factor for supporting vital QI; hemostasis by invigorating QI; invigorating the spleen and tonifying the kidney; softening the hard mass and removing blood stasis; resolving phlegm and eliminating dampness; relieving inflammation and evacuating toxin; promoting generation of vital essence and blood; promoting diuresis and invigorating vital QI; or the mixture thereof.

The culture medium useful for cultivation and proliferation of the present probiotics can be any known conventional culture medium containing a carbon source, a nitrogen source, and essential vitamins as well as minerals necessary for the bacteria growth. To minimize the cost for large-scale industrial production, the producing process is simplified and the culture medium is further supplemented with soybean isoflavone, soluble dietary fibers and soybean oligosaccharides, such as stachyose and raffinose, and the like. The culture medium consisting of bean sprout decocted liquor, enzymatic proteolytic beef broth, yeast extracts, carbohydrates, and minerals is preferred in the present invention. The culture medium used in the present invention is characterized by that it contains bean sprout decocted liquor which is about 25% v/v in the liquor medium, in addition to the other basic components, such as carbon sources (glucose and sucrose), nitrogen sources (enzymolytic broth and yeast extract) and minerals. A typical culture medium of the present invention consists of (by weight, w/w) 2.5% emzymolytic beef broth, 1.3% yeast extract, 25% soybean decocted liquor, 1.3% glucose, 1.3% sucrose, 0.05% $MgSO_4$, 0.05% $CaCO_3$, 0.03% $K_2HPO_4$, 0.03% $KH_2PO_4$, 0.03% NaCl.

It should be noted that the soybean sprout decocted liquor in the medium does not serve as a nitrogen and/or carbon source, but as the supplement of soybean isoflavone, soluble fibers and soybean oligosaccharides (such as stachyose and raffinose). It is known that soybean isoflavones as plant-derived estrogens including daizin, dye genitein, and daidzein, have some beneficial functions such as an anti-free radicals effect, reducing blood lipids level, lowering the risk of cardiovascular diseases, prevention of osteoporosis in menopausal woman caused by endocrine disturbance. The soluble fibers contained in said soybean sprout decocted liquor can interrupt the absorption of neutral lipid and cholesterol in the gut, promote gastro-intestine peristalsis, prevent constipation and delay or suppress the absorption of carbonhydrates. In addition, it is favorable to restore the balance between intestinal flora by said soybean decocted liquor because the soybean oligosaccharides contained therein can be used by intestinal *Lactobacillus* only, but almost not by harmful bacteria in the gut.

In conclusion, one characteristic of the present disclosure is the use of soybean decocted liquor as one of the main components in the culture medium for probiotics. It is determined that the content of soybean isoflavones and soluble fibers in the culture medium for probiotics according to the present disclosure is in a range of about 20-40 μg/ml and 30-100 μg/ml, respectively.

To prepare soybean decocted liquor, one of the main components in the culture medium of the present invention, fresh, and mature soybeans are soaked in two volume of warm water for about 5-6 hours until the tissue of the soybean has expanded. Said soybean is further maintained at a temperature of about 25-27 °C for around 60-72 hours until a sprout grows to 3-8 cm in length. The resultant sprouted and expended soybeans are collected and added into tap water in a ratio of 3 liters of water per liter of sprouted soybean. The mixture is then heated in an autoclave at about 105-121°C for 30min. After this, the soybean sprout decocted liquor is obtained by further filtration using a double-layer filter-cloth.

Another main component of the culture medium according to the present disclosure is the hydrolysate of protease-treated fresh beef and bovine liver tissue. The present broth is different from the conventional broth in that not only beef but also the bovine liver tissue which is rich in various enzymes are used. In addition, a row trypsin preparation obtained from porcine or bovine pancreas is also used during the process of making the present broth. To prepare the enzymolytic beef broth, selected beef and liver tissue are mixed in a ratio of 5: 2 (by weight) and then well minced. After that, 1-1.5 volumes of water are added into the chopped mixture, well mixed, and further heated to about 100 °C for about 60min. The temperature of said mixture cools down to 38-41 °C prior to the addition of the crude trypsin preparation made by the method describe below. The resultant is further digested for about 1 hour under 7-9. After hydrolysis, the proteolytic beef broth is obtained by collecting the supernatant after centrifugation at 2000 rpm.

The crude trypsin preparation is made from beef and bovine liver tissue according to the following steps: firstly, the pancreas from animals, such as bovine, equine, caprine or porcine are homogenated and then mixed with anhydrous ethanol and boiled water in a ration of 1:1:3, and further kept agitated for 72 hours. The supernatant is collected after centrifugation at 500 rpm for 10min before the pH of resulting supernatant is adjusted to about 5.5 by concentrated HCl. In this way, the desired crude trypsin preparation is obtained.

The water- or organic solution- extract of the natural medicines composition obtained by conventional techniques is added into the above-obtained culture medium as a matrix and thoroughly mixed. The resulting mixture comprising said matrix liquor and extract of natural medicines is heated to boiling for around 10 min, and then cooled to about 37 °C. At this temperature, said mixture is inoculated with one or more probiotics of human normal intestinal flora, which are selected from the group consisting of *Bifidobacterium, Lactobacillus, Streptococcus, Enterococcus, Propionium, Leuconostoc,* and *Bacillus.* Then the resultant is further fermented under about 37°C and pH 6.2 for around 18-24 hours. When the fermentation is over, the culture system is cooled to a room temperature and filtered before the natural medicines in the form of a liquor (i.e. filtrate) which is treated by probiotics fermentation is obtained.

Alternatively, according to the description in a co-pending application filed on the same day as the present application, one or more medicines from a natural source or the mixture thereof is/are incorporated into the total bacteria cultures at the appropriate time. In the above process, said medicines are converted or modified by the bacteria in the culture or by the metabolites thereof. Said suitable time for the treatment is between 24 hours from the end of probiotics co-fermentation and before the bacteria therein are killed by heating the whole fermentation system. After that, the resultant mixture is fermented for about another 18 hours under fermentation conditions that mimic the psychological condition, i.e., pH6.2, at 37°C.

During fermentation, one or more components in the extract of natural medicines will be modified structurally or converted metabolically by the anaerobic probiotics or the metabolites thereof. In this way, inactivated compounds of natural medicines will be converted into activated compounds, less activated molecules will be modified into the molecular or collections thereof with high biological activity, moreover, even some compounds with high toxic side-effect (especially animal-derived natural compound) will be converted into less toxic or non-toxic compounds or the collection thereof.

Although the application of *Lactobacillus acidophilus, Bifidobacterium bifidum* and *Enterococcus feacium* as probiotics in conversion of natural anti-cancer medicines has been described above, other culture mediums and fermentation protocols which make use of other components but still mimic the inter-intestinal environment may also useful to convert, for example, certain natural medicines under particular circumstances depending on the selected probiotics and the fermentation protocols.

In one of the examples of the present invention, a pharmaceutical composition as anti-cancer natural medicaments consisting of *Colla corii aisni* and extract of plant-derived components including *Radix astragal,* prepared *Radix rehmanniae, Radix codonopsis, Ganoderma lucidum, Rabdosia serra, Radix scutellariae,* honey-fried *Folium eriabotryae, Rhizoma phragmitis, Paris polyphylla*, etc., and animal-derived components including toadskin, *Bombyx batryticatus, Os eulotae* (snail), *Scorpio, Scolopendra* (Centipede), gecko etc., is preferred. The preparation of said composition will be discussed in example 2 in more details.

Surprisingly, no substantial effect on growth inhibition of cancer can be observed in triplicate experiments in which the above described anti-cancer natural medicines, without further fermentation with probiotics according to the present invention are directly administrated to the experimental S 180 sarcoma model animal or H22 hepatocarcinoma model animal, wherein the calculated inhibition rates are lower than 30%. Similarly, no detectable effect on growth inhibition of cancer can be observed when administrating the above described fermentation culture as they are, even when said culture comprises said three probiotics prepared according to the above described method as well as soybean isoflavones and soluble fibers. In another case, the above-mentioned water extract of plant- and animal-derived medicine is added into said fermentation culture in a ratio of 1:1, then the resulting mixture is fermented under anaerobic conditions at 39 °C±1 °C for about 18-25 hours. It is surprisingly found that the probiotics fermentation product of the extracts of total natural medicines prepared in this way exhibits substantial inhibition activity against the growth of cancer (see, examples 3 and 4).

Not wishing to be restricted by any established theory, the above result further demonstrates that natural medicines, especially some natural plant-and animal- derived medicines with known or unknown structure can be absorbed by the host only after certain chemical modification or bio-conversion by normal intestinal flora *in vivo* or *in vitro*, thereby their biological activity can be in function.

By way of example, examples 2 and 3 described below, details the process of treatment of a pharmaceutical composition comprising the extract of 13 kinds of plant derived component and 6 kinds of animal derived components by fermentation using three probiotics in the suitable nutrient culture medium.

In addition, living cancer harboring model animals are also used to demonstrate that the activity of said anti-cancer pharmaceutical composition is sharply enhanced after being fermented with intestinal probiotics of the present invention, compared with the same pharmaceutical composition that is not processed as such.

The resultant pharmaceutical composition of the present invention is useful as a potential aiding agent to the conventional anti-cancer treatment, including conventional chemical therapy and radiotherapy, and is also useful in reducing or eliminating to certain extent side-effects in the patient who is subject to the chemical therapy and/or radiotherapy (see, examples 4 and 5).

In another study on cancer-harboring mice, it is further proved that the anti-cancer natural medicines have the ability to enhance the immunological function in the cancer-loaded mice after being subjected to co-fermentation with probiocs according to the present invention. In particular, (1) the phagocytosis activity of macrophage cell in cancer-loaded mice is sharply enhanced; (2) the conversion of lymphocyte caused by mitotic factor of T cell, conA, is also improved (data not shown).

Thus, not being restricted to established theory, the present inventor found surprisingly that in addition to prophylaxis and treatment of various malignancy proliferative diseases, the Chinese traditional herbal medicaments of the present invention have the ability to promote the proliferation and development of hematopoietic stem cells in mammals, preferably in human beings.

Primary results of experiments on a cellular level as well as in animals, indicate that the neutrophils and erythrocytes level in the peripheral blood of a patient subjected to chemical therapy and/or radiotherapy can be restored and maintained effectively by administrating the preparation of the present invention: In addition, our primary experimental results suggest that in addition to being useful in remission of the inhibition of growth and differentiation of marrow stem cell caused by chemical therapy and/or radiotherapy, the present preparation is also useful in treating various leukemia and anemia.

The invention now being fully described in combination with the following examples and attached claims, it will be apparent to one of ordinary skill in the art that many changes and modifications can be made thereto without departing from the scope of the appended claims.

Examples

Example 1. Preparation of co-fermentation culture of intestinal protiotics

By way of illumination, the present example provides a method for preparation of a co-fermentation culture in same culture medium by inoculation in batches of *Lactobacillus acidophilus, Bifidobacterium bifidum*, and *Enterococcus faecium*, and co-fermentation.

1. Selection of the strains of probiotics

*Bifidobacterium* is one of the most important physiological bacteria in mammals, especially in human beings, which mainly distributes in the lower part of small intestine and colon. It is known that *Bifidobacterium breve* is very important to the nutrition, growth, development, and immunological function in humans. In the gasto-intestinal tract, most of said bacteria are located in the ileum. *Lactobacillus acidophilus*, is involved in biological synthesis of vitamins, especially vitamin B and will facilitate food digestion, promote nutrition metabolism as well as enhance the lactose tolerance of the host. *Enterococcus faecalis* is predominately located in the caecum, which facilitates metabolics of cholesterol and therefore reduces cholesterol level in the blood.

Thus, the selection of three protiotics, *Bifidobacterium breve, Lactobacillus acidophilus* and *Enterococcus faecalis,* in the present disclosure is based on the fact that they are all widely distributed in the human intestine and complementary to each other in their biological activity.

The three protiotics, *Bifidobacterium breve, Lactobacillus acidophilus* and *Enterococcus faecalis,* of the present invention are all available from China Center for Type Culture Collection (CCTCC), Wuhan, China. These bacteria can also be isolated from the feces or mucous membrane of the intestine in a healthy subject by conventional methods.

2. Composition of culture medium and the preparation of the same

The culture medium is composed of (per liter): 25ml proteolytic beef broth, 15 g yeast extract, 250ml soybean sprout decocted liquor, 12 g glucose, 13 g sucrose, 5g $MgSO_4$, 3g $CaCO_3$, 3g NaCl, 3g $K_2HPO_4$, and 3g $KH_2PO_4$.

The proteolytic beef broth and soybean sprout decocted liquor used in the culture medium of the present disclosure is prepared according to the above-described method in Details of Invention. The components in said culture medium are thoroughly mixed before being autoclaved at 121°C for 30 minutes and are then ready for use.

3. Method of co-fermentation by inoculation in batches

The 100ml of autoclaved culture medium according to the above description is heated to about 40 °C, and then inoculated by 1000g (wet weight) activate *Bifidobacterium breve* in a ratio of 0.8% (w/w) to the total culture weight calculated on the basis of the wet weight of biomass. Fermentation of the resulting mixture is carried out under anaerobic conditions at 39°C for 7.5 hour. When the pH value of the culture medium is reduced to 5.0, it is adjusted to about 6.2 by adding a suitable amount of 0.5N NaOH solution thereto while agitating, and further being inoculated with activate *Enterococcus faecalis* for over 20min while agitating. The amount of said *Enterococcus faecalis* is about 950 g (wet weight) calculated on biomass. The culture mixture is fermentated for another 6 hours at the same temperature. When the pH of said culture is again reduced to about 5.5, 0.5N NaOH solution is added and thereby the pH value is adjusted to 6.2, then *Lactobacillus acidophilus* in an amount of about 850 (wet weight) calculated in biomass is inoculated thereto and is kept fermenting at the same temperature. When the total bacteria content in the tank reaches more than 6.5 x $10^8$/ml with the pH value reduced to about 3.5-3.8, the temperature of the culture mixture is then cooled down to about 32°C by modulating the temperature of cycling water, and is maintained for about 2 hours so as to fulfill the biological conversion of the fermentation product.

Once the fermentation is finished the co-fermentation culture of probiotics obtained as the above description will be, depending on the particular purposes for application, (1) centrifugalized to collect living co-fermented probiotics before further lyophilized, thereby solid medicament or nutrition supplement containing lyophilized bacteria powders is obtained; (2) added into extracts of natural medicines to be converted, so as to obtain the preparation of the present invention having expected biological activity and function; or (3) heated to about 39 °C to kill proliferating bacteria therein, and optionally, be centrifugalized to collect supernatant, thereby nutrition supplement of the present invention is obtained which contains probiotics co-fermentation product and/or cell debris.

The results of biochemical analysis of the thus obtained supernatant of co-fermentation culture has shown that the content of hydrolyzed amino acid in the supernatant of the culture is up to 23.322 mg/ml, wherein disulfide bond containing cystine necessary for the growth of *Bifidobacterium* and *Lactobacillus* is 1.8 mg/ml (hydrolyzed) and 0.33 mg/ml (free form), respectively. The carbohydrate content in total in the supernatant of co-fermentation culture is about 0.04 mg/ml, wherein in addition to reduced sugars, oligosaccharide and diatery fiber essential to probiotics, such as *Bifidobacterium*, are presented. The latter in the intestine has functions of suppressing the level of blood sugar, preventing the absorption of neutral lipid and cholesterol as well as alleviating patients' suffering from constipation.

In addition, according to the results of ethyl acetate extraction and HPLC analysis, the bacteria converted product of soybean isoflavones, i.e., daidzin in the supernatant of said culture is up to 80 mg/ml. Furthermore, various vitamins (mostly vitamin B, about 40mg/l) and a certain amount of minerals (including P, Fe, Zn, Mg, K and Na as 5.04, 5.36, 8.29, 88.83, 783, 283 mg/ml, respectively) are also found in the supernatant of said culture.

The above listed results further indicate that the supernatant of co-fermentation culture can serve as a culture medium to provide all of the necessary nutrition for the growth of bacteria. The bacteria will not cease growth and/or proliferation until the pH value of the culture systems is reduced to lower than 4.0 as the result of the accumulation of acidic product brought by metabolic of bacteria with the growth of the bacteria.

Example 2: Preparation of extract of anti-cancer Chinese traditional herbs

By way of illumination, the present example describes a method for preparation of water extract of plant- and animal- derived medicines, respectively, depending on the original source of said medicines.

1. Preparation of extract of plant-derived medicine

Thirteen kinds of ingredients listed-below and processed conventionally in terms of Chinese traditional medical sciences are mixed in a 5 liter container, which include *Radix astragali* 12g, prepared *Radix rehmanniae* 20 g, *Radix codonopsis* 12g, *Ganoderma lucidum* 8g, *Radix trichosanthis* 12g, *Radiox glycyrrhizae* 5g, *Rabdosia serra*) 40g, *Radix scutellariae* 12 g, honey-fried *Folium eriabotryae* 12 g, *Rhizoma phragmitis* 12 g, *Paris polyphylla* 12g, *Semen juglandis* 20g, *Herba hedyotis diffusae* 12g. 1600 ml water is added into the resulting mixture (189 g in total). After the resultant mixture is decocted for about 1.5 hour, the first part of water extract (about 500ml) is therefore obtained by decantation. Then, another 1200ml water is added into the same container comprising the residue of the above decoction. The mixture of water and residue is decocted for about another 1.0 hour, the second part of water extract (about 400ml) is obtained similarly. Finally, 1200ml of water is again added into the same container being decocted in combination with the residues for about 0.5 hour, before the third part of the water extract is obtained. The three parts of water extracted are pooled and condensed under reduced pressure to about 300ml in total.

(2). Preparation of extract of animal-derived medicine

Six kinds of natural animal-derived medicines including toadskin 20g, *Bombyx batryticatus* 8g, *Os eulotae* (snail) 6g, *Scorpio* 4g, *Scolopendra* (Centipede) 4g, gecko 4g, which are processed by known methods in the art in the field of Chinese traditional medicines are added into a 2 liter container and mixed. Then 350ml water is added thereto and the mixture is soaked at room temperature for about 12 hours. After being minced by a tissue grinder at 1000 rpm for almost 2 minutes, the crushed mixture is decocted for about 15 hours. The water extract is decanted. Another 250 ml water is added thereto and then decocted for about 1 hour to obtain the second water extract. The two extracts are pooled and further filtered by a 4-layer filter-cloth. After cooling to room temperature, the resulting filtrate is added with 30g *Colla corii asini,* heated to about 80°C for 5 minutes till the added *Colla corii asini* melt, and then condensed under reduced pressure to 200 ml in total.

Example 3 Biological conversion to the extracts of anti-cancer natural medicines by intestinal probiotics

By way of illumination, the present example describes a method for biological conversion of the extract of natural medicines in two procedures (described below in details) by intestinal probiotics and/or fermentation product thereof.

1. The co-fermentation culture of *Bifidobacterium breve, Lactobacillus acidophilus* and *Enterococcus faecalis* is prepared according to the method described in example 1. When the total amount of bacteria in the culture reaches more than $7.0 \times 10^8$/ml, the culture is maintained in combination with living bacteria under 32°C for about 2 hours in order to substantially accomplish the biological conversion of said natural medicines. Then the whole culture mixture as a reaction system is heated to about 39°C and kept for about 6 hours to inactivate the bacteria therein. Optionally, the killed bacteria and large cell debris are removed by centrifugation (10000 x g, 20min). The natural plant-derived medicines and animal-derived medicines prepared according to the above-described methods are added into the resulting culture wherein the ratio of plant- and animal- derived medicines and culture is about 5:3: 2. The resultant mixture is reheated to 38-40°C, kept fermentating under anaerobic conditions for about 18 hours. When fermentation is finished, the culture medium is collected and centrifugated to separate the supernatant. Thus, the anti-cancer medicine converted by probiotics according to the present disclosure is obtained.
2. To the freshly made culture medium (25ml proteolytic beef broth, 15 g yeast extract, 250 ml soybean sprout decocted liquor, 12 g glucose, 13 g sucrose, 5g $MgSO_4$, 3g $CaCO_3$, 3g NaCl, 3g $K_2HPO_4$, and 3g $KH_2PO_4$) prepared as in Details of Invention described, aseptic extract of the above-described natural plant- and animal-derived medicines is added, wherein the ratio of culture medium, plant- and animal-derived medicines is 5:3:2. Once the extract and culture medium are thoroughly mixed, *Bifidobacterium breve, Lactobacillus acidophilus* and *Enterococcus faecalis* are inoculated in batches, i.e., in a same manner and amount as that described in example 1. The resultant mixture is fermented anaerobically at 38-40°C for about 20 hours. When fermentation is completed, the culture medium is collected and centrifugated to separate supernatant. Thus, the anti-cancer medicament converted by probiotics according to the present invention is obtained.

Example 4: Effect on the biological activity of extract of anti-cancer medicines by the fermentation conversion of intestinal probiotics

The present example relates to the improvement on the biological activity of the extract of anti-cancer natural medicines after its conversion *in vitro* by intestinal probiotics.

Using species Kunming mouse subcutaneously grafted S180 sacoma or H22 hepatocarcinoma as cancer-harboring model animal, the inhibition activity on growth of cancer is observed with respect to extract of anti-cancer natural medicines which are converted or unconverted by the above intestinal probiotics in either large dose or small dose.

For this purpose, 120 mice weighing about 18-25g are grafted subcutaneously at right axilla with $1 \times 10^7$ cells/ml of S180 sacoma or H22 hepatocarcinoma (0.2ml/mouse) in order to obtain solid tumor model animal. The resultant model animal are grouped randomly into 12 groups. Each animal in treatment group is orally administrated with unfermented (in 1:1 dilution) or fermented (in 1:4 dilution) extract of natural medicines daily.

Animals in negative control group are administrated same quantity of tap water, while in positive control group, animals are subcutaneously administrated with chemical synthetic anti-cancer agent, cyclophosphamide (CTX).

The administration of extract of natural medicine starts at the fourth day from the date of grafting cancer cells, i.e., the time when solid tumor can be touched by hand. After continuous administration over 10 days, the weight of animal and solid tumor are measured, respectively, and then inhibition rate of cancer can be calculated accordingly. The treatment experiments on the two types of cancer are carried out in triplicate. The experiment results on the S180 sacoma loaded animal model or H22 hepatocarcinoma loaded animal model are listed in table 1 and table 2, respectively.

Table 1 The result of effect on the inhibition activity on S180 sacoma loaded mouse model with respect to the extract of anti-cancer medicaments converted or unconverted by the co-fermentation culture of intestinal probiotics

| groups (n=10) | animal weight | | solid tumor weight ($X\pm SD$) | inhibition rate (%) | P value |
|---|---|---|---|---|---|
| | before ad. | after ad. | | | |
| negative control | 20.9* | 26.1 | 1.41±0.69 | | |
| positive control | 20.1 | 21.2 | 0.55±0.19 | 61.0 | <0.001 |
| converted extract | | | | | |
| large dose | 20.4 | 23.9 | 0.99±0.36 | 38.1 | <0.05 |
| small dose | 20.6 | 25.9 | 1.44±1.18 | 21.3 | <0.05 |
| unconverted extract | | | | | |
| large dose | 21.0 | 23.8 | 0.93±0.49 | 29.5 | >0.05 |
| small dose | 20.6 | 24.6 | 1.22±0.89 | -4.1 | >0.05 |
| * the listed data are the means of the results of experiments in triplicate standard error ($X\pm SD$) | | | | | |

Table 2: The result of effect on the inhibition activity on H22 hepatocarcinoma loaded mouse model with respect to the extract of anti-cancer medicaments converted or unconverted by co-fermentation culture of intestinal probiotics

| groups (n=10) | animal weight | | solid tumor weight($X\pm SD$) | inhibition rate (%) | P value |
|---|---|---|---|---|---|
| | before ad. | after ad. | | | |
| negative control | 21.2* | 28.8 | 1.91±0.78 | | |
| positive control | 21.2 | 27.9 | 0.72±0.36 | 62.4 | <0.001 |
| converted extract | | | | | |
| large dose | 20.6 | 28.6 | 1.80±0.47 | 44.8 | <0.001 |
| small dose | 21.0 | 30.4 | 1.07±0.53 | 26.2 | <0.05 |
| unconverted extract | | | | | |
| large dose | 21.6 | | 1.14±0.38 | 29.6 | >0.05 |
| small dose | 21.5 | 27.4 | 1.45±0.49 | 10.5 | >0.05 |
| * the listed data are the means of the results of experiments in triplicate standard error ($X\pm SD$) | | | | | |

In the treatment experiment, if the inhibition rate of a tested sample is higher than 30%, said sample is considered having anti-cancer (or cancer inhibition) activity, if not, said sample has no anti-cancer (or cancer inhibition) activity. According to the data listed in tables 1 and 2, not subjecting biological conversion by intestinal normal flora or metabolites thereof, the extract of plant- and animal- derived medicines exhibit no statistically significant anti-cancer (or cancer inhibition) activity, whose inhibition rate is in the range of 10.5-29.5% only. Surprisingly, after co-fermentation with the intestinal probiotics of the present invention, the biological activity of the extract according to the present invention is substantially enhanced, i.e., inhibition rate is higher than 40%, due to the biochemical structure modification and enzymatic conversion by probiotics and metabolites thereof.

Example 5. The effect of anti-cancer natural medicines preparation which is converted and modified by intestinal probiotics and metabolites thereof on the conventional chemical therapy and radiotherapy for cancer

In the present example, by using the above described cancer-harboring mouse model, the following results are obtained.

(1) The synergetic effect of the fermented natural medicine preparation on the conventional chemical therapy and radiotherapy for cancer is observed when the present preparation is administrated accompanied by conventional chemical therapy and radiotherapy; and
(2) The ability of the present preparation in respect to the prevention or relief of the toxicity or side effects caused by conventional chemical and radiotherapy is also confirmed.

1. For the above purpose, 80 mice (half male, half female) weighing about 18-22g are grafted subcutaneously at right axilla with suspension of H22 hepatocarcinoma in saline (1:3 dilution, $1 \times 10^7$ cells/ml, 0.2ml/mouse) in order to obtain solid tumor model mouse. The resultant model mouse is grouped randomly into eight groups.

Each mouse in first four groups is treated for observing the effect of the present natural medicine preparation on the efficiency of anti-cancer chemical agent, fliorouracil (5-Fu, 15 mg/kg), mice in the rest four groups is for the effect of the present compositions on the efficiency of radiotherapy for cancer ($^{60}$Co irradiation on whole body, 4.5 Gray /day/ animal).

The combination therapy to both animal groups (4 subgroups in each group) starts from the next day of grafting tumor cells and continues for 7 days. In particular, animals in subgroup 2 are administrated with the present preparation; and animals of subgroup 4 are treated with the present preparation in combination with administration of anti-cancer chemical agents or $^{60}$Co irradiation (treatment group); while the animals in subgroup 3 are injected subcutaneously with either anti-cancer chemical agent or subjected to $^{60}$Co irradiation (positive control group); and animals in subgroup 1 are injected with equal volume water or subjected mild dosage of $^{60}$Co irradiation (negative control group).

After continuous administration over 7 days, the weight of the animal and solid tumor are measured, respectively, and then inhibition rate of cancer can be calculated accordingly. The synergetic effect of the present composition on chemical therapy or radiotherapy for cancer is calculated by the inhibition rate according to the following Burgi's correction equation:

$$Q = E(A+B)/EA+EB-EAxEB$$

wherein, E (A+B) is the cancer inhibition rate of the combined therapy, EA and EB are the cancer inhibition rate of the present composition administration, or the chemical therapy (or radiotherapy) alone.

Result: (1) If Q calculated is lower than 0.85, it is considered as an antergenic effect between combined therapy; (2) if Q is between 0.85 and 1.15, it is considered as an addictive effect; and (3) if Q is above 1.15, then it is deemed as synergetic effect between combined therapy. The particular results are listed in table 3 and table 4, respectively.

Table 3 The result of therapy effect of the present preparation in combination with anti-cancer chemical agents on the cancer-harboring mouse (H22 hepatocarcinoma)

| groups (n=10) | animal weight | | tumor weight (X±SD) | inhibition rate (%) | P value |
|---|---|---|---|---|---|
| | before ad. | after ad. | | | |
| 1 | 20.5 | 29.5 | 2.15±0.67 | | |
| 2 | 20.9 | 25.9 | 0.72±0.21 | 66.5 | <0.001 |
| 3 | 20.9 | 27.4 | 1.06±0.31 | 56.7 | <0.001 |
| 4 | 21.2 | 23.5 | 0.39±0.18 | 81.9 | <0.001* |
| * Q= 0.99, so there is an addictive effect on the cancer-loaded mouse (H22 hepatocarcinoma) by administration of the present preparation in combined with 5-Fu. | | | | | |

Table 4 The result of therapy effect of the present preparation in combination with radiotherapy ($^{60}$Co irradiation) on the cancer-loaded mouse (H22 hepatocarcinoma)

| groups (n=10) | animal weight | | tumor weight (X±SD) | inhibition rate (%) | P value |
|---|---|---|---|---|---|
| | before ad. | after ad. | | | |
| 1 | 21.7 | 33.3 | 2.77±0.29 | | |
| 2 | 21.4 | 31.1 | 1.31±0.13 | 52.7 | <0.01 |
| 3 | 21.5 | 24.2 | 0.99±0.05 | 64.3 | <0.001 |
| 4 | 21.9 | 25.6 | 0.62±0.06 | 77.6 | <0.001* |
| * Q= 0.93, so there is an addictive effect on the cancer-loaded mouse (H22 hepatocarcinoma) by administration of the present preparation in combined with $^{60}$Co irradiation. | | | | | |

Concluded from the data listed in table 3 and 4, the effect of combination therapy by the present anti-cancer preparation together with conventional chemical therapy or radiotherapy for cancer is greatly superior to that of a single therapy alone in terms of cancer inhibition rate. In addition, such a combination therapy has an additive effect deduced from the Q value thereof which is in the range between 0.85- 1.95.

2. On the other hand, 80 mice (half male, half female) weighed from about 18-22g are grouped randomly into two groups with four subgroups in each group.

Animals in treatment group (subgroups 3 and 4) are gavaged daily with medium dose or small dose of the anti-cancer preparation according to the present invent, for a continuous 7 days, and simultaneously (1) anti-cancer chemical agent, cyclophosphamide (CTX), is administrated to subjected animals in single dosage (60 mg/Kg) on the first day (see, table 5); or (2) $^{60}$Co irradiation (4.5 Gray) is carried out daily for 7 continuous days.

Animals in negative control group (subgroup 1) are provided with equal volume water, while in positive group (subgroup 2) subjected equivalent dosage of chemical agent or radiotherapy alone. 7 days later, the content of haemoglobin and counting of white blood cell (WBC), red blood cell (RBC) and platelet (BPC) are determined after sampling peripheral blood from caudal vein. The results are shown in tables 5 and 6.

Table 5 The result of protect effect of the present preparation on the hemogram damage in peripheral blood caused by chemical therapy

| groups (n=10) | WBC (x $10^6$/ml) | WBC (x $10^9$/ml) | WBC (x $10^6$/ml) | WBC (x $10^6$/ml) |
|---|---|---|---|---|
| 1 | 8.78±3.75 | 8.91±1.50 | 130.0±24.0 | 15.6±1.3 |
| 2 | 4.28±1.48 | 6.70±1.98 | 56.5±23.0 | 13.7±0.8 |
| 3 | 6.62±1.44 | 9.90±1.43 | 118.5±26.0 | 17.6±0.7 |
| 4 | 5.56±1.83 | 9.44±1.22 | 128.5±35.7 | 16.9±0.6 |

Table 6 The result of protect effect of the present preparation on the hemogram damage in peripheral blood caused by radiotherapy

| groups(n=10) | WBC (x $10^6$/ml) | WBC (x $10^9$/ml) , | WBC (x $10^6$/ml) | WBC (x $10^6$/ml) |
|---|---|---|---|---|
| 1 | 7.64±1.95 | 8.59±1.03 | 158.7±32.6 | 15.5±0.6 |
| 2 | 0.64±0.34 - | 7.65±1.27 | 163.5±36.4 | 13.8±0.8 |
| 3 | 1.42±1.14 | 8.08±1.35 | 165-.0±74.2 | 17.1±1.1 |
| 4 | 1.25±0.36 | 7.13±1.64 | 164.1±64.7 | 16.7±1.1 |

Both chemical agent, CTX, and $^{60}$ Co irradiation will destroy the hematopoietic system in the subject animal and result in the destruction of WBC, RBC, Hb, and BPC, wherein the amount of WBC and the Hb content are strongly effected by radiotherapy. It is shown by the results listed in tables 5 and 6 that the said damage caused by radiotherapy or chemical therapy can be effectively eliminated or ameliorated by the present preparation processed by the fermentation of probiotics according to the present invention.

Example 6: Effect of present preparation on the proliferation of human bone marrow cell

By way of illumination, the present example describes the effect of the present preparation on the proliferation of human bone marrow cell, wherein said composition is converted and modified by the intestinal probiotics and metabolites thereof.

The cell culture of non-adhering human bone marrow cell with T cell depleted is plated onto agar plate supplemented with 15% human serum albumin in an amount of 40,000 cell/plate, then added with 0.5ml present preparation. The plate is incubated under 5% $CO_2$, 5% $O_2$ atmosphere for 10 days. When incubation is finished, the number of cell colony is accounted using invert microscope and the results are expressed in colony formation unit as CFU-GEMM for granulocyte, red blood cell, monocyte, and macrophage colony; as CFU-GM for granulocyte-macrophage colony; and as CRU-E for red blood cell colony.

The background of each plate without supplement of natural medicine composition of the present invent or cytokine (granulocyte colony stimulation factor, G-CSF) is determined by adding 1 unit epoetin thereto and then counting the colony unit of red blood cell. The commercially available G-CSF (1000 pg/ml) and GM-GSF (1200 pg/ml) are used as positive control, PBS buffer (1ml) as negative control. The result is shown is table 7.

Table 7 Result of the effect of natural medicine composition on the proliferation of human bone marrow cell

| agent | dosage (pg/ml) | number of the cell colony (means $\pm$ S.D.) | | |
|---|---|---|---|---|
| | | CFU-GEMM | CFU-GM | CFU-E |
| blank control | | 2$\pm$ | 0 | 45$\pm$4 |
| composition | 1ml | 7$\pm$0.5 | 20$\pm$2 | 60$\pm$5 |
| G-CSF | 1000 | 8$\pm$1 | 25$\pm$3 | 68$\pm$3 |
| GM-CSF | 1000 | 8.5$\pm$0.5 | 28$\pm$1 | 48$\pm$2 |

**Claims**

1. A process for producing preparations of natural medicines comprising the following steps:

   - inoculating one or more intestinal probiotics into a culture medium which contains the extract of the medicines; and
   - culturing the same under suitable fermentation conditions,
   - wherein the intestinal probiotics are one or more human intestinal non-pathogenic probiotics that are selected from the group of *Bifidobacterium, Lactobacillus, Streptococcus, Enterococcus, Propionium, Leuconostoc,* and *Bacillus.*

2. The process according to claim 1,
   comprising the following steps:

   - preparing a co-fermentation culture of one or more intestinal probiotics;
   - subsequently adding into the culture medium a pre-made extract of one or more natural medicines or the powder of non-processed natural medicines; and
   - subsequently mixing and culturing the resultant mixture.

3. The process according to claim 1,
   comprising the following steps:

   (a) adding into the suitable culture medium a pre-made extract of one or more natural medicines or the powder of non-processed natural medicines;
   (b) subsequently inoculating in batches one or more intestinal probiotics into the mixture obtained in step (a) and culturing the same by co-fermentation.

**4.** The process according to claim 1,
wherein the probiotics are selected from the species of the group consisting of *Bifidobacterium, bifidum, Bifidobacterium breve, Bifidobacterium infantis, Bifidobacterium longum, Lactobacillus acidophilus, Lactobacillus bulgaricus, Lactobacillus casei, Lactobacillus delbrueckii, Lactobacillus plantarum, Streptococcus thermophilus, Enterococcus faecalis, Enterococcus faecium, Leuconostoc citrovorum, Propionibacterium shermanii*, *Bacillus subtilis,* and *Bacillus licheniformis.*

**5.** The process according to claim 1,
wherein the preparations of natural medicines comprise one or more of any plant or animal originating medicaments or combinations thereof having prophylaxis and/or treatment effect, and which can be processed or modified by one or more normal human intestinal bacteria and metabolites thereof.

**6.** The process according to claim 1,
wherein the preparations of natural medicines are the water-extract or organic-solution extract of one or more natural medicines or the combination thereof, or the non-processed solid powders of the medicines.

**7.** The process according to claim 6,
wherein the organic solution is selected from the group consisting of methanol, ethanol, n-propanol, isopropanol, methylether, ethyl ether, dimethylether, acetone, dichloromethane, chloroform, acetonitrile, or the mixture thereof.

**8.** The process according to claim 1,
wherein the preparations of natural medicines are selected from the group consisting of medicaments for eliminating pathogenic factor for supporting vital QI; hemostasis by invigorating QI; invigorating the spleen and tonifying the kidney; softening the hard mass and removing blood stasis; resolving phlegm and eliminating dampness; relieving inflammation and evacuating toxin; promoting generation of blood; promoting diuresis and invigorating vital QI; or selected from a mixture of such medicaments.

**Patentansprüche**

**1.** Verfahren zum Herstellen von Präparaten von Naturarzneimitteln,
das die folgenden Schritte aufweist:

- Impfen von einem intestinalen Probiotikum oder mehreren intestinalen Probiotika in ein Kulturmedium, das den Extrakt der Arzneimittel enthält; und
- Züchten derselben unter geeigneten Fermentierungsbedingungen,

wobei die intestinalen Probiotika ein oder mehrere intestinale nicht-pathogene Human-Probiotika sind, die aus der Gruppe ausgewählt sind, die aus *Bifidobacterium, Lactobacillus, Streptococcus, Enterococcus, Propionium, Leuconostoc* und *Bacillus* besteht.

**2.** Verfahren nach Anspruch 1,
das die folgenden Schritte aufweist:

- Vorbereiten einer Cofermentationskultur von einem intestinalen Probiotikum oder mehreren intestinalen Probiotika;
- anschließendes Zugeben von einem vorgefertigten Extrakt von einem oder mehreren Naturarzneimitteln oder dem Pulver von unverarbeiteten Naturarzneimitteln in das Kulturmedium; und
- anschließendes Mischen und Züchten des entstandenen Gemischs.

**3.** Verfahren nach Anspruch 1,
das die folgenden Schritte aufweist:

(a) Zugeben von einem vorgefertigten Extrakt von einem oder mehreren Naturarzneimitteln oder dem Pulver von unverarbeiteten Naturarzneimitteln in das geeignete Kulturmedium;
(b) anschließendes chargenweises Impfen von einem intestinalen Probiotikum oder mehreren intestinalen Probiotika in das im Schritt (a) erhaltene Gemisch und Züchten desselben durch Cofermentation.

**4.** Verfahren nach Anspruch 1,
wobei die Probiotika aus den Spezies der Gruppe ausgewählt sind, die aus *Bifidobacterium bifidum, Bifidobacterium breve, Bifidobacterium infantis, Bifidobacterium longum, Lactobacillus acidophilus, Lactobacillus bulgaricus, Lactobacillus casei, Lactobacillus delbrueckii, Lactobacillus plantarum, Streptococcus thermophilus, Enterococcus faecalis, Enterococcus faecium, Leuconostoc citrovorum, Propionibacterium shermani, Bacillus subtilis* und *Bacillus licheniformis* besteht.

**5.** Verfahren nach Anspruch 1,
wobei die Präparate von Naturarzneimitteln eines oder mehrere aus von irgendeiner pflanze oder irgendeinem Tier stammenden Arzneimitteln oder Kombinationen davon mit Prophylaxe- und/oder Behandlungswirkung aufweisen und durch irgendein oder mehrere normale Human-Darmbakterien und Stoffwechselprodukte davon behandelt oder modifiziert werden können.

**6.** Verfahren nach Anspruch 1,
wobei die Präparate von Naturarzneimitteln der wäßrige Extrakt oder ein Extrakt einer organischen Lösung von einem oder mehreren Naturarzneimitteln oder der Kombination davon oder die unverarbeiteten festen Pulver der Arzneimittel sind.

**7.** Verfahren nach Anspruch 6,
wobei die organische Lösung aus der Gruppe ausgewählt ist, die aus Methanol, Ethanol, n-Propanol, Isopropanol, Methylether, Ethylether, Dimethylether, Aceton, Dichlormethan, Chloroform, Acetonitril oder dem Gemisch davon besteht.

**8.** Verfahren nach Anspruch 1,
wobei die Präparate von Naturarzneimitteln aus der Gruppe ausgewählt sind, die aus folgendem besteht: Arzneimitteln für die Eliminierung des pathogenen Faktors, um das vitale QI zu unterstützen, für die Hämostase durch Kräftigung des QI, für die Kräftigung der Milz und Verbesserung des Tonus der Nieren, für das Weichmachen der harten Masse und das Entfernen eines Blutstaus, für das Auflösen eines Phlegmas und das Beseitigen der Feuchtigkeit, für die Abschwächung einer Entzündung und die Beseitigung von Toxin, für die Förderung der Blutbildung, für die Förderung von Diurese und die Kräftigung des vitalen QI;
oder aus einem Gemisch solcher Arzneimittel ausgewählt sind.

**Revendications**

**1.** Procédé pour produire des préparations de médicaments naturels, comprenant les étapes suivantes:

- inoculation d'un ou plusieurs probiotiques intestinaux dans un milieu de culture qui contient l'extrait des médicaments; et
- culture de ce(s) dernier(s) dans des conditions de fermentation appropriées,
- dans lequel les probiotiques intestinaux sont un ou plusieurs probiotiques intestinaux humains non pathogènes qui sont choisis dans le groupe de *Bifidobacterium, Lactobacillus, Streptococcus, Enterococcus, Propionium, Leuconostoc,* et *Bacillus.*

**2.** Procédé selon la revendication 1,
comprenant les étapes suivantes:

- préparation d'une culture de co-fermentation d'un ou plusieurs probiotiques intestinaux;
- puis ajout dans le milieu de culture d'un extrait pré-fait d'un ou plusieurs médicaments naturels ou de la poudre de médicaments naturels non-traités; et
- puis mélange et culture du mélange résultant.

**3.** Procédé selon la revendication 1,
comprenant les étapes suivantes:

(a) ajout dans le milieu de culture approprié d'un extrait pré-fait d'un ou plusieurs médicaments naturels ou de la poudre de médicaments naturels non-traitées;
(b) puis inoculation par lots d'un ou plusieurs probiotiques intestinaux dans le mélange obtenu dans l'étape (a) et culture de ce(s) dernier(s) par la co-fermentation.

4. Procédé selon la revendication 1,
dans lequel les probiotiques sont choisis parmi les espèces du groupe constitué par *Bifidobacterium bifidum, Bifidobacterium breve, Bifidobacterium infantis, Bifidobacterium longum, Lactobacillus acidophilus, Lactobacillus-bulgaricus, Lactobacillus casei, Lactobacillus delbrueckii, Lactobacillus plantarum, Streptococcus thermophilus, Enterococcus faecalis, Enterococcus faecium, Leuconostoc citrovorum, Propionibacterium shermanii, Bacillus subtilis* et *Bacillus licheniformis.*

5. Procédé selon la revendication 1,
dans lequel les préparations de médicaments naturels comprennent un ou plusieurs médicaments provenant de tout(e) plante ou animal ou des combinaisons de ceux-ci ayant un effet dans la prophylaxie et/ou le traitement, et qui peuvent être traités ou modifiés par une ou plusieurs bactéries intestinales humaines normales et des métabolites de celles-ci.

6. Procédé selon la revendication 1,
dans lequel les préparations de médicaments naturels sont l'extrait à l'eau ou l'extrait par une solution organique d'un ou plusieurs médicaments naturels ou la combinaison de ceux-ci, ou les poudres solides non-traités des médicaments.

7. Procédé selon la revendication 6,
dans lequel la solution organique est choisie dans le groupe constitué par le méthanol, l'éthanol, le n-propanol, l'isopropanol, le méthyléther, l'éther éthylique, le diméthyléther, l'acétone, le dichlorométhane, le chloroforme, l'acétonitrile, ou le mélange de ceux-ci.

8. Procédé selon la revendication 1,
dans lequel les préparations de médicaments naturels sont choisies dans le groupe constitué par les médicaments pour éliminer un facteur pathogène pour soutenir le QI vital; hémostase par stimulation du QI; stimulation de la rate et tonification du rein; ramollissement de la masse dure et enlèvement de la stase du sang; résolution du flegme et élimination de l'humidité; soulagement de l'inflammation et évacuation de toxine; induction de la génération du sang; induction de la diurèse et stimulation du QI vital; ou choisis parmi un mélange de tels médicaments.

## REFERENCES CITED IN THE DESCRIPTION

**Patent documents cited in the description**

- CN 1232682, JIA Y

**Non-patent literature cited in the description**

- **Dooth A.N. et al.** *J. Biol.Chem.,* 1956, vol. 223, 251
- **Drasar ; Hill.** Human Intestinal Flora. Academic press, 1974
- **Scheline R.K.** *Pharmacol. Rev.,* 1973, vol. 25, 451
- **Niissou, A. et al.** *Biochem. Biophys. Acta,* 1967, vol. 148, 92